# EUROPEAN PATENT APPLICATION

(11) **EP 4 652 927 A1**
(43) Date of publication of application: **26.11.2025**
(21) Application number: 25171770.8
(22) Date of filing: 22.04.2025
(51) Int. Cl.: A61B 5/11, A61B 5/00, G06N 20/00, G16H 20/30, G16H 50/70

(54) **METHOD TO ESTABLISH DETERMINATION MODEL AND METHOD TO DETERMINE FOOT ACCESSORY**

(30) Priority: 24.05.2024 TW 113119330
(71) Applicant: Imotek Inc., 404 Taichung City (TW)
(72) Inventor: CHANG, Ching-Wei, 100 Taipei City (TW)
(74) Representative: DREISS Patentanwälte PartG mbB

(57) **Abstract**

A method to establish a determination model for determining a foot accessory is to be implemented by an electronic device. The electronic device stores training data sets that correspond to sampled objects. The method includes: for each of the training data sets, grouping entries of sensor data of the training data set into sensor-data groups that correspond to phases of a specific activity; establishing a classification model based on the entries of sensor data that belong to a target one of the sensor-data groups of each of the training data sets; and combining the classification model and a lookup table by using an output of the classification model as an input of the lookup table so as to obtain the determination model.

## Description

The disclosure relates to a method to establish a determination model for determining a foot accessory, and a method to determine a foot accessory.

Different people may have different foot shapes and different gaits, and thus different kinds of foot accessories are required to fit their feet. Conventionally, a person usually tries on the foot accessory to determine whether or not the foot accessory fits his/her feet. However, such approach may be unreliable, and an improper foot accessory may make the person uncomfortable, cause the person to have a poor posture, impose an additional burden on the person, and even increase the risk of getting injured to the person.

Therefore, an object of the disclosure is to provide a determination model for determining a foot accessory for a specific type of movement of a subject, and a method to determine a foot accessory for a specific type of movement of a subject that can alleviate at least one of the drawbacks of the prior art.

According to the aforementioned two aspects of the disclosure, the methods are provided as set out in each of the appended claims.

Other features and advantages of the disclosure will become apparent in the following detailed description of the embodiment(s) with reference to the accompanying drawings. It is noted that various features may not be drawn to scale.
Figure 1 is a block diagram illustrating a user device and a sensor device according to an embodiment of the disclosure.
Figure 2 is a flow chart illustrating a method to establish a determination model according to an embodiment of the disclosure.
Figure 3 is a schematic diagram illustrating a gait cycle of a stance phase of walking.
Figure 4 is a schematic diagram illustrating an example of forward/backward accelerations against inward/outward accelerations for three types of movement.
Figure 5 is a flow chart illustrating a method to determine a foot accessory according to an embodiment of the disclosure.

Before the disclosure is described in greater detail, it should be noted that where considered appropriate, reference numerals or terminal portions of reference numerals have been repeated among the figures to indicate corresponding or analogous elements, which may optionally have similar characteristics.

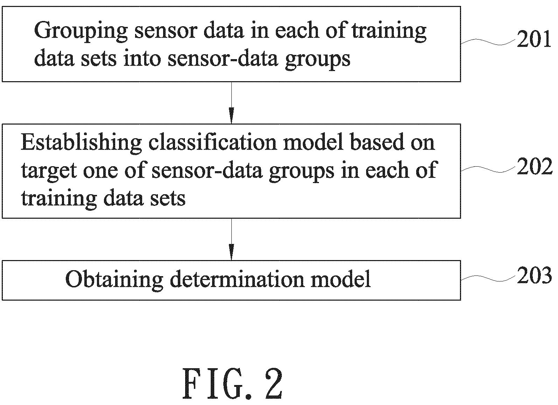

Referring to Figure 2, an embodiment of a method to establish a determination model for determining a foot accessory for a specific type of movement of a subject (e.g., a human) according to the disclosure is illustrated. The foot accessory may be implemented to include a pair of insoles, a pair of shoes, a pair of socks, an assistive device for feet and so on, but is not limited thereto.

The method is to be implemented by an electronic device (not shown). The electronic device may be implemented by a smartphone or a personal computer (e.g., a tablet computer, a desktop computer, a notebook computer and so on), but is not limited thereto.

The electronic device stores a plurality of training data sets that correspond respectively to a plurality of sampled objects. The sampled objects are people other than the subject. Each of the training data sets is related to one of a plurality of specific types of movement. Each of the training data sets contains, for the corresponding one of the sampled objects, a plurality of entries of sensor data that were generated based on detection on movement of the sampled object and that were respectively at a plurality of time spots when the sampled object was performing a specific activity. The entries of sensor data were generated by a measuring device that is in communication with the electronic device and that was mounted on a lower limb (e.g., a thigh, a calf or a foot) of the corresponding one of the sampled objects of each of the training data sets. The measuring device may be implemented to include an inertial measurement unit (IMU) such as one or more of a gyroscope for measuring orientation and angular velocity or an accelerometer for sensing proper acceleration, a magnetometer for measuring magnetic field or magnetic dipole moment, etc. The communication between the electronic device and the measuring device may be implemented to involve the Bluetooth^{®} wireless technology, the Wi-Fi technology, radio-frequency identification (RFID) and so on. In a scenario where an anterior/posterior direction, a superior/inferior direction and a medial/lateral direction of a lower limb are defined respectively as three dimensional axes in the three-dimensional Cartesian coordinate systems, the entries of sensor data generated by the measuring device may include an angular velocity of rotation of an axis of the lower limb that is measured by the gyroscope, and an acceleration of the lower limb that is sensed by the accelerometer and that includes three acceleration components corresponding respectively with the dimensional axes, and three relative changes of the magnetic field around the lower limb with respect to the dimensional axes, respectively.

The specific activity has a plurality of phases. In this embodiment, the specific activity is walking, and the specific activity has a stance phase and a swing phase. It is worth to note that the stance phase is a state where a foot under consideration is in contact with a floor for supporting, and the swing phase is a state where the foot under consideration is not in contact with the floor (i.e., a leg connected to the foot is swinging in the air) for moving. The specific types of movement to which the training data sets are related may include types of over-pronation, pronation and supination. However, the specific activity and the phases thereof are not limited to the disclosure herein and may vary in other embodiments.

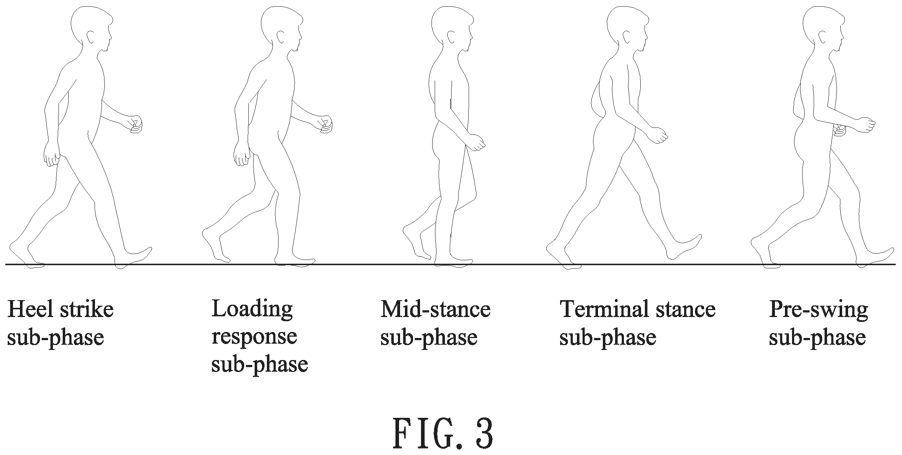

Referring to Figure 3, it is worth to note that in one embodiment, a gait cycle of the stance phase of walking can be further divided into a heel strike sub-phase, a loading response sub-phase, a mid-stance sub-phase, a terminal stance sub-phase and a pre-swing sub-phase. The heel strike sub-phase is related to a situation that a heel of a foot of a human makes initial contact with the ground, and the human slightly bends his/her knee to prepare for supporting his/her body weight. The loading response sub-phase is related to a situation that the human keeps bending his/her knee, and the foot rolls forward until the entire sole of the foot is in contact with the ground to support his/her body weight. The mid-stance sub-phase is related to a situation that the human gradually extends his/her knee, and the body weight of the human is propelled forward so that the greater trochanter of femur of the human is directly above the middle of the foot. The terminal stance sub-phase is related to a situation that the human lifts his//her heel off the ground. The pre-swing sub-phase is related to a situation that the human pushes his/her toes into the ground and bends his/her knee, so as to create a forward propulsion. During the gait cycle of the stance phase of walking, knee flexion achieves a maximal degree at the latter stage of the pre-swing sub-phase.

In one embodiment, the specific activity is riding a bicycle, and the specific activity has a top-dead-center (TDC) phase, a powering phase, a bottom-dead-center (BDC) phase and a recovery phase. It is worth to note that the TDC phase is a state where the bicycle is pedaled by a cycler to make a crank of the bicycle pass through a top dead center of the crank, the powering phase is a state where the cycler applies force (i.e., extends his/her leg to produce a down-stroke) to propel the bicycle forward, the BDC phase is a state where the bicycle is pedaled by the cycler to make the crank of the bicycle pass through a bottom dead center of the crank, and the recovery phase is a state where the cycler lifts his/her leg during a transition from the BDC phase to the TDC phase.

The method to establish the determination model includes steps 201 to 203 as shown in Figure 2 and delineated below.

In step 201, for each of the training data sets, the electronic device groups the entries of sensor data of the training data set into a plurality of sensor-data groups that correspond respectively to the phases of the specific activity. In this embodiment, the electronic device groups the entries of sensor data of the training data set that are related to the stance phase into a stance-phase group, and groups the entries of sensor data of the training data set that are related to the swing phase into a swing-phase group. More specifically, the electronic device utilizes a machine learning algorithm to obtain the stance-phase group and the swing-phase group based on features (e.g., changes of accelerations in direction or extremes of angular velocities) derived from the entries of sensor data and based on criteria that are related to the features and that are determined in advance for distinguishing the stance-phase group and the swing-phase group. Since implementation of grouping the entries of sensor data of the training data set into the stance-phase group and the swing-phase group has been well known to one skilled in the relevant art, detailed explanation of the same is omitted herein for the sake of brevity.

In the embodiment where the specific activity is riding a bicycle, the electronic device groups the entries of sensor data that are related to the TDC phase into a TDC-phase group, groups the entries of sensor data that are related to the powering phase into a powering-phase group, groups the entries of sensor data that are related to the BDC phase into a BDC-phase group, and groups the entries of sensor data that are related to the recovery phase into a recovery-phase group.

In step 202, based on the entries of sensor data that belong to a target one of the sensor-data groups of each of the training data sets, the electronic device establishes a classification model for determining one of the specific types of movement of the subject and for outputting said one of the specific types of movement as an output. In this embodiment, the target one of the sensor-data groups is one of the stance-phase group and the swing-phase group. In the embodiment where the specific activity is riding a bicycle, the target one of the sensor-data groups is one of the TDC-phase group, the powering-phase group, the BDC-phase group and the recovery-phase group.

It should be noted that in this embodiment, the electronic device establishes the classification model directly based on the entries of sensor data that belong to the target one of the sensor-data groups. In other embodiment, for each of the training data sets, the electronic device performs data processing on the entries of sensor data that belong to the target one of the sensor-data groups to generate a plurality of entries of processed training data that respectively correspond to the entries of sensor data. Each of the entries of processed training data contains one of a movement trajectory that is related to a limb segment of a lower limb of the corresponding one of the sampled objects, a moving velocity that is related to the lower limb of the corresponding one of the sampled objects, and a swept area that is related to swing of the lower limb of the corresponding one of the sampled objects. Thereafter, the electronic device establishes the classification model based on the entries of processed training data of each of the training data sets.

In some embodiments, each of the training data sets further contains a physiological parameter that is related to the corresponding one of the sampled objects, and the electronic device establishes the classification model further based on the physiological parameter contained in each of the training data sets. The physiological parameter is exemplarily an age, a sex, a body weight, a foot shape, a leg shape or the like.

The way to establish the classification model may be implemented by three approaches delineated below.

In a first approach, for each of the specific types of movement, the electronic device generates a reference probability distribution based on the entries of sensor data that belong to the target one of the sensor-data groups of each of the training data sets (each of the training data sets is related to the specific type of movement). Then, the electronic device establishes the classification model that includes the reference probability distributions respectively for the specific types of movement. The reference probability distribution is exemplarily a normal distribution, but is not limited thereto.

In a second approach, each of the training data sets further contains a label that indicates one of the specific types of movement to which the training data set is related. For example, one of the training data sets related to the type of over-pronation would contain a label that indicates the type of over-pronation, one of the training data sets related to the type of pronation would contain a label that indicates the type of pronation, and one of the training data sets related to the type of supination would contain a label that indicates the type of supination. The electronic device uses a machine learning algorithm to establish the classification model further based on the label contained in each of the training data sets. Since implementation of using a machine learning algorithm to establish the classification model has been well known to one skilled in the relevant art, detailed explanation of the same is omitted herein for the sake of brevity.

In a third approach, for each of the entries of sensor data belonging to the target one of the sensor-data groups of each of the training data sets, the electronic device obtains an inward/outward acceleration and a forward/backward acceleration based on the entry of sensor data. It is worth to note that the inward acceleration represents an acceleration for a medial portion of a lower limb of a human moving toward a longitudinal axis of a trunk of the human, the outward acceleration represents an acceleration for a lateral portion of the lower limb of the human moving away from the longitudinal axis of the trunk of the human, the forward acceleration represents an acceleration of the lower limb in a forward direction of the human when the human bends his/her knee to lift the lower limb, and the backward acceleration represents an acceleration of the lower limb in a backward direction of the human when the human straightens his/her knee to extend the lower limb. The inward/outward acceleration and the forward/backward acceleration can be directly obtained by the accelerometer of the measuring device. Since utilizing the measuring device to obtain the inward/outward acceleration and the forward/backward acceleration has been well known to one skilled in the relevant art, detailed explanation of the same is omitted herein for the sake of brevity. For example, please refer to publications such as "Body CoM acceleration for rapid analysis of gait variability and pedestrian effects on structures" authored by Chiara Bedon in 2022, "Wearable inertial sensors to measure gait and posture characteristic differences in older adult fallers and non-fallers: A scoping review" authored by Patel et al. in 2020, "An innovative approach of using inertial sensor data to detect abnormal gait patterns" authored by Patterson et al. in 2012, or "A review of accelerometry-based wearable motion detectors for physical activity monitoring" authored by Yang et al. in 2010.

For each of the specific types of movement, the electronic device determines a reference inward/outward interval based on the inward/outward accelerations and the forward/backward accelerations that are obtained based on the entries of sensor data of the training data sets which are related to the specific type of movement. More specifically, for each of the specific types of movement, an interval-determining procedure is executed as follows. Firstly, the electronic device selects a plurality of desired forward/backward accelerations from among the forward/backward accelerations thus obtained for the entries of sensor data, wherein the desired forward/backward accelerations thus selected are relatively greater among the forward/backward accelerations. For example, the desired forward/backward accelerations are top ten percent of the forward/backward accelerations, but are not limited thereto. Secondly, the electronic device selects a plurality of desired inward/outward accelerations from among the inward/outward accelerations, wherein the desired inward/outward accelerations are obtained based on a part of the entries of sensor data, based on which the desired forward/backward accelerations are obtained. Thirdly, the electronic device designates a range of the desired inward/outward accelerations as the reference inward/outward interval.

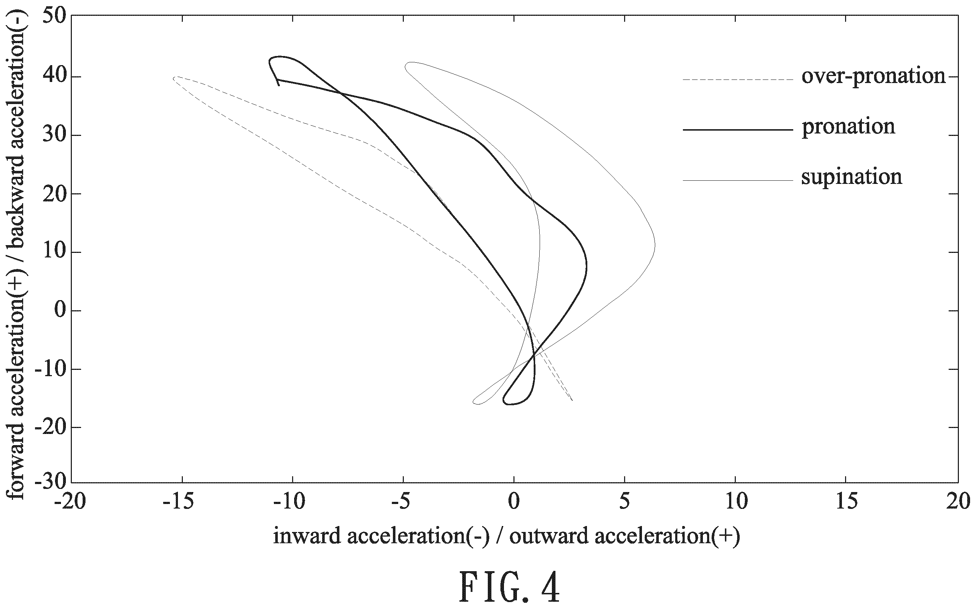

Figure 4 illustrates an example of forward/backward accelerations against inward/outward accelerations for the types of over-pronation, pronation and supination. In the figure, a minus sign specifies a forward/backward acceleration as a backward acceleration, and a plus sign specifies a forward/backward acceleration as a forward acceleration. In addition, a minus sign specifies an inward/outward acceleration as an inward acceleration, a plus sign specifies an inward/outward acceleration as an outward acceleration. For the type of over-pronation, the desired inward/outward accelerations are obtained based on a part of the entries of sensor data, based on which the desired forward/backward accelerations (i.e., the top ten percent greatest ones of the forward/backward accelerations) are obtained, and are -16, -15, -14 and -13 m/s²; a range of the desired inward/outward accelerations, i.e., from -16 to -13 m/s², would be designated as the reference inward interval. Similarly, for the type of pronation, the desired inward/outward accelerations are obtained based on a part of the entries of sensor data, based on which the desired forward/backward accelerations (i.e., the top ten percent greatest ones of the forward/backward accelerations) are obtained, and are -11, -10 and -9 m/s²; a range of the desired inward/outward accelerations, i.e., from -11 to -9 m/s², would be designated as the reference inward interval. Likewise, for the type of supination, the desired inward/outward accelerations are obtained based on a part of the entries of sensor data, based on which the desired forward/backward accelerations (i.e., the top ten percent greatest ones of the forward/backward accelerations) are obtained, and are -4 and -3 m/s²; a range of the desired inward/outward accelerations, i.e., from -4 to -3 m/s², would be designated as the reference inward interval.

In a scenario where the stance phase of walking is further divided into the heel strike sub-phase, the loading response sub-phase, the mid-stance sub-phase, the terminal stance sub-phase and the pre-swing sub-phase, in order to distinguish the specific types of movement according to the entries of sensor data that are collected at the latter stage of the pre-swing sub-phase, the reference inward/outward intervals are determined respectively for the specific types of movement by executing the aforementioned interval-determining procedure based on the entries of sensor data thus collected.

After the reference inward/outward intervals have been determined respectively for the specific types of movement, the electronic device establishes the classification model that includes the reference inward/outward intervals.

In other embodiments, for each of the entries of sensor data belonging to the target one of the sensor-data groups of each of the training data sets, the electronic device obtains an angular velocity based on the entry of sensor data. For each of the specific types of movement, the electronic device determines an angular-velocity criterion (which may be a threshold or an interval) based on the angular velocities that are obtained based on the entries of sensor data of the training data sets which are related to the specific type of movement. Then, the electronic device establishes the classification model that includes the angular-velocity criteria determined for the specific types of movement.

In step 203, the electronic device obtains the determination model for determining a foot accessory for the subject based on the output of the classification model, wherein the determination model is configured to associate with the specific types of movement with a plurality of kinds of foot accessories (e.g., a pair of insoles, a pair of shoes, a pair of socks, an assistive device for feet, etc.).

For example, the electronic device further stores a lookup table that contains the specific types of movement and the kinds of foot accessories that are related respectively to the specific types of movement. The electronic device combines the classification model and the lookup table by using the output of the classification model as an input of the lookup table so as to obtain the determination model for determining one of the kinds of foot accessories for the subject.

It is worth to note that for the type of over-pronation, a foot of a person would extremely roll inward upon landing while the person is walking, which causes a fallen-arch, so a supportive insole to stabilize the arch would be suitable for the person with the type of over-pronation. For the type of pronation, a foot of a person would normally roll inward upon landing while the person is walking, so an insole with medium arch support would be suitable for the person with the type of pronation. For the type of supination, a foot of a person would roll outward upon landing while the person is walking and the high arch need a buffer for less impact, so an insole with excessive strain and cushioning would be suitable for the person with the type of supination.

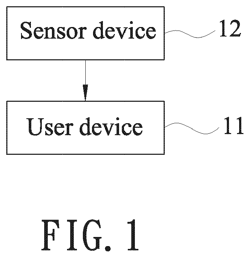

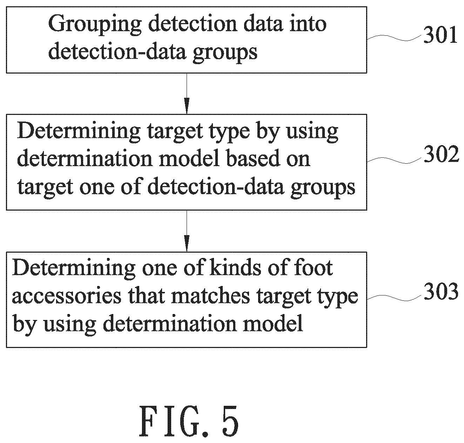

Referring to Figure 5, an embodiment of a method to determine a foot accessory for the specific type of movement of the subject (i.e., a human) according to the disclosure is illustrated. The method is to be implemented by a user device 11 as shown in Figure 1. The user device 11 is electrically connected to a sensor device 12 that is mounted on a lower limb of the subject. The sensor device 12 generates, based on detection, a plurality of entries of detection data that were respectively at a plurality of time spots when the subject was performing the specific activity. The user device 11 stores the determination model that has been established by the electronic device previously described, and that is for determining, for the subject, one of a plurality of kinds of foot accessories (the kinds of foot accessories are related respectively to the specific types of movement).

The user device 11 may be implemented by a smartphone or a personal computer (e.g., a tablet computer, a desktop computer, a notebook computer and so on), but is not limited thereto. The sensor device 12 may be implemented to include the IMU and the magnetometer. The electrical connection between the user device 11 and the sensor device 12 may be implemented to involve the Bluetooth^{®} wireless technology, the Wi-Fi technology, RFID and so on. It should be noted that the user device 11 may be implemented to be identical to or different from the electronic device that is previously described, and the sensor device 12 may be implemented to be identical to or different from the measuring device that is previously described.

The method includes steps 301 to 303 delineated below.

In step 301, the user device 11 groups the entries of detection data into a plurality of detection-data groups that correspond respectively to the phases of the specific activity. Specifically, in this embodiment, the user device 11 groups the entries of detection data that are related to the stance phase into a stance-phase group and groups the entries of detection data that are related to the swing phase into a swing-phase group. A target one of the detection-data groups is one of the stance-phase group and the swing-phase group.

In the embodiment where the specific activity is riding a bicycle, the user device 11 groups the entries of detection data that are related to the TDC phase into a TDC-phase group, groups the entries of detection data that are related to the powering phase into a powering-phase group, groups the entries of detection data that are related to the BDC phase into a BDC-phase group, and groups the entries of detection data that are related to the recovery phase into a recovery-phase group. A target one of the detection-data groups is one of the TDC-phase group, the powering-phase group, the BDC-phase group and the recovery-phase group.

In step 302, the user device 11 determines, by using the determination model based on the entries of detection data that belong to the target one of the detection-data groups, one of the specific types of movement that is related to the subject as a target type.

It should be noted that in this embodiment, the user device 11 determines the target type by using the classification model directly based on the entries of detection data that belong to the target one of the detection-data groups. In other embodiment, the user device 11 performs data processing on the entries of detection data that belong to the target one of the detection-data groups to generate a plurality of entries of processed detection data that respectively correspond to the entries of detection data. Each of the entries of processed detection data contains one of a movement trajectory that is related to a limb segment of a lower limb of the subject, a moving velocity that is related to the lower limb of the subject, and a swept area that is related to swing of the lower limb of the subject. Thereafter, the user device 11 determines the target type by using the determination model based on the entries of processed detection data.

In some embodiments where the classification model is established further based on the physiological parameters that are related respectively to the sampled objects, the user device 11 determines the target type by using the determination model further based on a physiological parameter (e.g., an age, a sex, a body weight, a foot shape, a leg shape or the like) that is related to the subject.

The way to determine the target type by using the classification model may be implemented by three approaches delineated below.

In a first approach, the determination model includes the reference probability distributions respectively for the specific types of movement. The user device 11 generates a detected probability distribution based on the entries of detection data that belong to the target one of the detection-data groups. Then, the user device 11 selects one of the reference probability distributions that is most similar to the detected probability distribution as a target distribution, and determines the target type that corresponds to the target distribution.

In a second approach, the classification model is established by using a machine learning algorithm further based on the label. The user device 11 feeds the entries of detection data that belong to the target one of the detection-data groups into the classification model, and obtains an output of the classification model as the target type.

In a third approach, the determination model includes the reference inward/outward intervals respectively for the specific types of movement. For each of the entries of detection data that belong to the target one of the detection-data groups, the user device 11 obtains an inward/outward acceleration and a forward/backward acceleration based on the entry of detection data. The user device 11 selects a plurality of target forward/backward accelerations from among the forward/backward accelerations thus obtained for the entries of detection data, where the target forward/backward accelerations thus selected satisfy a statistical feature of the forward/backward accelerations. In this embodiment, the statistical feature is that the target forward/backward accelerations are the top ten percent greatest ones of the forward/backward accelerations. Moreover, the user device 11 selects a plurality of target inward/outward accelerations from among the inward/outward accelerations, where the target forward/backward accelerations are obtained based on a part of the entries of detection data, based on which the target forward/backward accelerations are obtained. Afterwards, the user device 11 calculates an average of the target inward/outward accelerations, and selects one of the reference inward/outward intervals within which the average of the target inward/outward accelerations falls as a target interval. The user device 11 determines the target type that corresponds to the target interval.

Referring to Figure 4, in a scenario where the reference inward interval for the type of over-pronation is from -16 to -13 cm/s², the reference inward interval for the type of pronation is from -11 to -9 cm/s², and the reference inward interval for the type of supination is from -4 to -3 cm/s², when the user device 11 calculates an average of the target inward/outward accelerations to be -13 cm/s², the user device 11 would determine the reference inward interval from -16 to -13 cm/s² as the target interval and determine the type of over-pronation as the target type.

In one embodiment where the classification model includes the angular-velocity criteria, for each of the entries of detection data belonging to the target one of the detection-data groups, the user device 11 obtains an angular velocity based on the entry of detection data. The user device 11 selects a plurality of target angular velocities from among the angular velocities thus obtained for the entries of detection data, where the target angular velocities thus selected satisfy a statistical feature of the angular velocities. In this embodiment, the statistical feature is that the target angular velocities are the top ten percent greatest ones of the angular velocities, but is not limited thereto. After that, the user device 11 calculates an average of the target angular velocities, and selects one of the angular-velocity criteria with which the average of the target angular velocities matches (e.g., the average of the target angular velocities falls within an interval of the one of the angular-velocity criteria) as a target criterion. The user device 11 determines the target type that corresponds to the target criterion.

In step 303, the user device 11 determines one of the kinds of foot accessories that matches the target type by using the determination model.

To sum up, for the method to establish a determination model and the method to determine a foot accessory, the entries of sensor data of the training data set are grouped into the sensor-data groups at first according to the phases of the specific activity, and then the classification model is established based on the target one of the sensor-data groups. In this way, a specific type of movement for a subject could be determined by using the classification model. It is worth to note that computational resources for establishing the classification model may be saved and robustness of the classification model may be improved. Furthermore, the classification model is combined with the lookup table to obtain the determination model, and a foot accessory for the subject could be properly and reliably determined by using the determination model.

In the description above, for the purposes of explanation, numerous specific details have been set forth in order to provide a thorough understanding of the embodiment(s). It will be apparent, however, to one skilled in the art, that one or more other embodiments may be practiced without some of these specific details. It should also be appreciated that reference throughout this specification to "one embodiment," "an embodiment," an embodiment with an indication of an ordinal number and so forth means that a particular feature, structure, or characteristic may be included in the practice of the disclosure. It should be further appreciated that in the description, various features are sometimes grouped together in a single embodiment, figure, or description thereof for the purpose of streamlining the disclosure and aiding in the understanding of various inventive aspects; such does not mean that every one of these features needs to be practiced with the presence of all the other features. In other words, in any described embodiment, when implementation of one or more features or specific details does not affect implementation of another one or more features or specific details, said one or more features may be singled out and practiced alone without said another one or more features or specific details. It should be further noted that one or more features or specific details from one embodiment may be practiced together with one or more features or specific details from another embodiment, where appropriate, in the practice of the disclosure.

## Claims

1. A method to establish a determination model for determining a foot accessory for a specific type of movement of a subject, the method to be implemented by an electronic device, the electronic device storing a plurality of training data sets that correspond respectively to a plurality of sampled objects, each of the training data sets being related to one of a plurality of specific types of movement, and containing, for the corresponding one of the sampled objects, a plurality of entries of sensor data that were generated based on detection on movement of the sampled object and that were respectively at a plurality of time spots when the sampled object was performing a specific activity, the specific activity having a plurality of phases, the method **characterized by**:
for each of the training data sets, grouping the entries of sensor data of the training data set into a plurality of sensor-data groups that correspond respectively to the phases of the specific activity;
based on the entries of sensor data that belong to a target one of the sensor-data groups of each of the training data sets, establishing a classification model for determining one of the specific types of movement of the subject and for outputting said one of the specific types of movement as an output; and
obtaining the determination model for determining the foot accessory for the subject based on the output of the classification model, the determination model being configured to associate with the specific types of movement with a plurality of kinds of foot accessories.

2. The method as claimed in claim 1, the specific activity being walking and having a stance phase and a swing phase,
wherein grouping the entries of sensor data of the training data set into a plurality of sensor-data groups includes grouping the entries of sensor data of the training data set that are related to the stance phase into a stance-phase group and grouping the entries of sensor data of the training data set that are related to the swing phase into a swing-phase group, and, in establishing the classification model, the target one of the sensor-data groups is one of the stance-phase group and the swing-phase group.

3. The method as claimed in claim 1, the specific activity being riding a bicycle and having a top-dead-center "TDC" phase, a powering phase, a bottom-dead-center "BDC" phase and a recovery phase,
wherein grouping the entries of sensor data of the training data set into a plurality of sensor-data groups includes grouping the entries of sensor data that are related to the TDC phase into a TDC-phase group, grouping the entries of sensor data that are related to the powering phase into a powering-phase group, grouping the entries of sensor data that are related to the BDC phase into a BDC-phase group, and grouping the entries of sensor data that are related to the recovery phase into a recovery-phase group, and, in establishing the classification model, the target one of the sensor-data groups is one of the TDC-phase group, the powering-phase group, the BDC-phase group and the recovery-phase group.

4. The method as claimed in any one of claims 1 to 3, further comprising:
for each of the specific types of movement, generating a reference probability distribution based on the entries of sensor data that belong to the target one of the sensor-data groups of each of the training data sets, the training data sets being related to the specific type of movement,
wherein establishing a classification model is to establish the classification model that includes the reference probability distributions respectively for the specific types of movement.

5. The method as claimed in any one of claims 1 to 3, each of the training data sets further containing a label that indicates the one of the specific types of movement to which the training data set is related,
wherein establishing a classification model includes using a machine learning algorithm to establish the classification model based on the label contained in each of the training data sets.

6. The method as claimed in any one of claims 1 to 3, further comprising:
for each of the entries of sensor data of each of the training data sets, obtaining an inward/outward acceleration and a forward/backward acceleration based on the entry of sensor data; and
determining a plurality of reference inward/outward intervals respectively for the specific types of movement based on the inward/outward accelerations and the forward/backward accelerations thus obtained respectively for the entries of sensor data of each of the training data sets,
wherein establishing a classification model is to establish the classification model that includes the reference inward/outward intervals.

7. The method as claimed in any one of claims 1 to 6, further comprising:
for each of the training data sets, performing data processing on the entries of sensor data that belong to the target one of the sensor-data groups to generate a plurality of entries of processed training data that respectively correspond to the entries of sensor data,
wherein each of the entries of processed training data contains one of a movement trajectory that is related to a limb segment of a lower limb of the corresponding one of the sampled objects, a moving velocity that is related to the lower limb of the corresponding one of the sampled objects, and a swept area that is related to swing of the lower limb of the corresponding one of the sampled objects, and
wherein establishing a classification model includes establishing the classification model based on the entries of processed training data of each of the training data sets.

8. The method as claimed in any one of claims 1 to 7, each of the training data sets further containing a physiological parameter that is related to the corresponding one of the sampled objects,
wherein establishing a classification model includes establishing the classification model based on the physiological parameter contained in each of the training data sets.

9. A method to determine a foot accessory for a specific type of movement of a subject, to be implemented by a user device (11), the user device (11) being electrically connected to a sensor device (12) that is mounted on a lower limb of the subject, the sensor device (12) generating, based on detection, a plurality of entries of detection data that were respectively at a plurality of time spots when the subject was performing a specific activity, the specific activity having a plurality of phases, the user device (11) storing a determination model that is obtained using the method of Claim 1, the method **characterized by**:
grouping the entries of detection data into a plurality of detection-data groups that correspond respectively to the phases of the specific activity;
determining, by using the determination model based on the entries of detection data that belong to a target one of the detection-data groups, one of a plurality of specific types of movement that is related to the subject as a target type; and
determining one of a plurality of kinds of foot accessories that matches the target type by using the determination model.

10. The method as claimed in claim 9, wherein the determination model includes a plurality of reference probability distributions respectively for the specific types of movement, the method further comprising:
generating a detected probability distribution based on the entries of detection data that belong to the target one of the detection-data groups; and
selecting one of the reference probability distributions that is most similar to the detected probability distribution as a target distribution,
wherein determining a target type is to determine the target type that corresponds to the target distribution.

11. The method as claimed in claim 9, wherein the determination model includes a plurality of reference inward/outward intervals respectively for the specific types of movement, the method further comprising:
for each of the entries of detection data that belong to the target one of the detection-data groups, obtaining an inward/outward acceleration and a forward/backward acceleration based on the entry of detection data;
selecting a plurality of target forward/backward accelerations from among the forward/backward accelerations thus obtained for the entries of detection data, the target forward/backward accelerations thus selected satisfying a statistical feature of the forward/backward accelerations;
selecting a plurality of target inward/outward accelerations from among the inward/outward accelerations, the target forward/backward accelerations being obtained based on a part of the entries of detection data, based on which the target forward/backward accelerations are obtained;
calculating an average of the target inward/outward accelerations; and
selecting one of the reference inward/outward intervals within which the average of the target inward/outward accelerations falls as a target interval,
wherein determining a target type is to determine the target type that corresponds to the target interval.

12. The method as claimed in any one of claims 9 to 11, further comprising:
performing data processing on the entries of detection data that belong to the target one of the detection-data groups to generate a plurality of entries of processed detection data that respectively correspond to the entries of detection data,
wherein each of the entries of processed detection data contains one of a movement trajectory that is related to a limb segment of the lower limb of the subject, a moving velocity that is related to the lower limb of the subject, and a swept area that is related to swing of the lower limb of the subject, and
wherein determining a target type is to determine the target type by using the determination model based on the entries of processed detection data.

13. The method as claimed in any one of claims 9 to 12, wherein determining a target type is to determine the target type by using the determination model further based on a physiological parameter that is related to the subject.

14. The method as claimed in any one of claims 9 to 13, the specific activity being walking and having a stance phase and a swing phase,
wherein grouping the entries of detection data into a plurality of detection-data groups includes grouping the entries of detection data that are related to the stance phase into a stance-phase group and grouping the entries of detection data that are related to the swing phase into a swing-phase group, and, in determining a target type, the target one of the detection-data groups is one of the stance-phase group and the swing-phase group.

15. The method as claimed in any one of claims 9 to 13, the specific activity being riding a bicycle and having a top-dead-center "TDC" phase, a powering phase, a bottom-dead-center "BDC" phase and a recovery phase,
wherein grouping the entries of detection data into a plurality of detection-data groups includes grouping the entries of detection data that are related to the TDC phase into a TDC-phase group, grouping the entries of detection data that are related to the powering phase into a powering-phase group, grouping the entries of detection data that are related to the BDC phase into a BDC-phase group, and grouping the entries of detection data that are related to the recovery phase into a recovery-phase group, and, in determining a target type, the target one of the detection-data groups is one of the TDC-phase group, the powering-phase group, the BDC-phase group and the recovery-phase group.
